# FASCICULE DE BREVET EUROPEEN

(11) **EP 2 362 772 B1**
(45) Date de publication et mention de la délivrance du brevet: **23.01.2013**
(21) Numéro de dépôt: 09768124.1
(22) Date de dépôt: 02.11.2009
(51) Int. Cl.: A61K 31/194, A61K 31/385, A61P 35/00, A61K 31/14, A61K 31/165, A61K 31/444, A61K 31/714, A61K 33/24

(54) **ASSOCIATION PHARMACEUTIQUE CONTENANT L'ACIDE LIPOÏQUE ET L'ACIDE HYDROXYCITRIQUE A TITRE DE PRINCIPES ACTIFS**
PHARMAZEUTISCHE VERBINDUNG WELCHE LIPONSÄURE UND HYDROXYCITRONENSÄURE ALS AKTIVSUBSTANZEN ENtHALTEN
PHARMACEUTICAL ASSOCIATION CONTAINING LIPOIC ACID AND HYDROXYCITRIC ACID AS ACTIVE INGREDIENTS

(30) Priorité: 03.11.2008 FR 0857448
(43) Date de publication de la demande: 07.09.2011
(73) Titulaire: Biorebus, 75008 Paris (FR); Schwartz, Laurent, 75006 Paris (FR)
(72) Inventeur: SCHWARTZ, Laurent, F-75006 Paris (FR); GUAIS-VERGNE, Adeline, F-91210 Draveil (FR)
(74) Mandataire: Hubert, Philippe
(86) Numéro de dépôt international: PCT/FR2009/052110
(87) Numéro de publication internationale: WO 2010/061088

(56) Documents cités:
- WO-A-00/48594
- WO-A-2004/100885
- US-A1- 2002 132 219
- US-A1- 2005 095 233
- US-B2- 7 335 384

## Description

La présente invention a pour objet une nouvelle association pharmaceutique comprenant, à titre de principes actifs, l'acide lipoïque ou l'un de ses sels pharmaceutiquement acceptables et l'acide hydroxycitrique ou l'un de ses sels pharmaceutiquement acceptables.

Cette association pharmaceutique, qui peut se présenter sous forme unitaire ou sous forme de kit, présente une activité antitumorale particulièrement élevée.

L'acide lipoïque est un co-facteur de plusieurs complexes enzymatiques.

L'acide lipoïque est également un antioxydant puissant. Il aide dans la protection des cellules contre les dommages des radicaux libres.

Ce produit est connu, en tant que principe actif de médicament. Il est en particulier préconisé pour le traitement de neuropathies associées au diabète, de myopathies mitochondriales et de scléroses multiples.

Il est parfaitement toléré et sa toxicité est très faible. A titre d'exemple, il peut être administré en une quantité comprise entre 600 et 1800 mg/j.

L'utilisation de l'acide lipoïque ou de l'un de ses sels solubles dans l'eau, seul ou en association avec l'acide ascorbique, a été préconisée dans le traitement du cancer, notamment par le document WO 00/48594 correspondant au brevet US 6 448 287.

Par ailleurs, l'utilisation de dérivés de l'acide lipoïque ou de leurs sels pharmaceutiquement acceptables a également été préconisée dans le traitement des maladies néoplasiques par le document WO 00/24734 correspondant au brevet US 6 951 887.

Cependant, aucun médicament à base d'acide lipoïque ou de l'un de ses dérivés ou de l'un de leurs sels pharmaceutiquement acceptables ne semble à ce jour en cours de développement pour le traitement du cancer.

L'acide hydroxycitrique est un produit naturel que l'on trouve à l'état naturel dans l'écorce des fruits du tamarinier de malabar (Garcinia). Son sel de calcium (l'hydroxycitrate de calcium) est connu pour inhiber la biosynthèse des acides gras.

L'utilisation de l'hydroxycitrate de calcium a ainsi été préconisée pour la perte de poids, en association avec une alimentation faible en gras, la posologie recommandée étant de 500 mg à 1 500 mg trois fois par jour avant les repas.

C'est une substance parfaitement bien tolérée chez l'adulte et chez l'enfant.

Parallèlement, l'hydroxycitrate de calcium est connu pour augmenter l'oxydation des acides gras au niveau des cellules hépatiques, ce qui permet leur transformation en glycogène. Le glycogène est ensuite stocké au niveau des muscles pour être disponible en cas d'effort. L'hydroxycitrate est ainsi utilisé dans de nombreux régimes diététiques pour le traitement de l'obésité. Il présente notamment l'avantage de ne pas modifier le taux de glucose sanguin. Le brevet US 6,207,714 souligne que l'hydroxycitrate peut être utilisé comme hypoglycémiant pour le traitement de personnes atteintes de diabète résistant à l'insuline.

Par ailleurs, l'hydroxycitrate est cité parmi les très nombreux composés susceptibles d'être utilisés dans le traitement de cellules cancéreuses présentant un taux élevé de glycolyse aérobie (document WO 2004/100885).

Dans ce contexte, il a été découvert, et ceci constitue le fondement de la présente invention, que l'association de l'acide lipoïque ou de l'un de ses sels pharmaceutiquement acceptables et de l'acide hydroxycitrique ou de l'un de ses sels pharmaceutiquement acceptables présente une activité antitumorale particulièrement élevée résultant d'un effet synergique de ses principes actifs constitutifs.

Il a en particulier été démontré que cette nouvelle association permet de limiter la croissance des tumeurs d'une façon tout à fait inattendue, le volume desdites tumeurs se stabilisant sur une durée d'au moins 100 jours à des valeurs sensiblement égales au volume des tumeurs en début de traitement. Cet effet de stabilisation des tumeurs est de façon surprenante, supérieur à celui obtenu à l'aide de médicaments anticancéreux connus.

Par conséquent, l'association faisant l'objet de la présente invention est particulièrement originale du fait de la synergie potentialisatrice des actions de chacun de ces principes actifs.

Ainsi, selon un premier aspect, la présente demande vise à couvrir une association pharmaceutique qui comprend :
- d'une part, l'acide lipoïque ou l'un de ses sels pharmaceutiquement acceptables ; et
- d'autre part, l'acide hydroxycitrique ou l'un de ses sels pharmaceutiquement acceptables lesdits ingrédients étant formulés ensemble ou séparément pour une utilisation, dans le traitement des tumeurs.

Par ailleurs, l'acide lipoïque présente 1 atome de carbone assymétriques. Il peut donc exister sous forme d'énantiomères ou de diastéréoisomères. Ces énantiomères et diastéréosiomères ainsi que leurs mélanges, y compris les mélanges racémiques, font partie de l'invention. D'une manière préférée, on utilisera la forme R de l'acide lipoïque.

D'une façon générale, les deux principes actifs caractérisant l'association pharmaceutique selon l'invention peuvent être formulés ensemble (forme unitaire) ou séparément (kit).

Egalement d'une façon générale, les deux principes actifs, formulés ensemble ou séparément, peuvent être administrés simultanément ou séparément avec un décalage dans le temps qui peut être souhaitable pour une optimisation de leur action conjuguée au vu de la nature de leur formulation respective.

Un sel pharmaceutiquement acceptable de l'acide lipoïque peut être un sel soluble dans l'eau tel que décrit dans le brevet US 6 448 287.

Un sel pharmaceutiquement acceptable de l'acide hydroxycitrique peut être un sel de métal alcalin (notamment de sodium) ou alcalino-terreux (notamment de calcium ou de magnésium).

Les associations pharmaceutiques de l'invention comprennent les deux principes actifs identifiés précédemment. Selon un mode de réalisation particulier, elles ne comprennent aucun autre principe actif. Alternativement, la présence d'au moins un autre principe actif au sein de ces nouvelles associations peut être envisagée.

Ainsi, il a été montré que l'efficacité des associations pharmaceutiques de l'invention comprenant les deux principes actifs identifiés précédemment peut être améliorée lorsque ces deux principes actifs sont associés à au moins un principe actif additionnel choisi dans le groupe constitué du cisplatine, de la capsaïcine, de la choline, de la miltéfosine et de la vitamine B12.

Des associations pharmaceutiques multiples préférées dans ce contexte sont notamment :
- les associations triples constituées des deux principes actifs identifiés précédemment et d'un principe actif additionnel choisi parmi le cisplatine et la capsaïcine ;
- les associations multiples constituées :
- soit des deux principes actifs identifiés précédemment, de la capsaïcine et du cisplatine ;
- soit des deux principes actifs identifiés précédemment, de la capsaïcine, de la miltéfosine, de la choline et de la vitamine B12.

Selon un premier mode de réalisation actuellement préféré de l'invention, l'association pharmaceutique précitée consiste en une forme unitaire incorporant les principes actifs habituellement dans un excipient pharmaceutiquement acceptable.

Selon un second mode de réalisation, cette association pharmaceutique se présente sous forme d'un kit contenant :
- d'une part, l'acide lipoïque ou l'un de ses sels pharmaceutiquement acceptables, habituellement dans un excipient pharmaceutiquement acceptable ; et
- d'autre part, l'acide hydroxycitrique ou l'un de ses sels pharmaceutiquement acceptables, habituellement dans un excipient pharmaceutiquement acceptable.

Quel que soit le mode de réalisation envisagé, la nature et les quantités respectives des différents excipients pourront être facilement déterminées par un homme du métier en fonction de la forme galénique finale souhaitée.

De façon préférée, dans le cas d'une forme unitaire, les principes actifs seront conditionnés sous une forme galénique adaptée à une administration orale. Toutefois, d'autres voies d'administration comme par exemple les voies intramusculaire, intraveineuse, topique ou cutanée peuvent être envisagées.

De même, d'une façon préférée, dans le cas où les principes actifs se présentent séparément, ils seront conditionnés indépendamment l'un de l'autre, chacun sous une forme galénique adaptée à l'administration par voie orale. Toutefois, d'autres voies d'administration peuvent être envisagées pour chacune des deux formes galéniques, et de façon indépendante.

Selon une caractéristique particulière, une forme galénique adaptée à la voie orale peut être choisie parmi les comprimés, les gélules, les poudres, les granulés, les lyophilisats, les solutés buvables et les sirops.

Les comprimés constituent cependant la forme galénique adaptée à la voie orale actuellement préférée. Ces comprimés peuvent être de nature variée, à libération immédiate, contrôlée ou retardée et éventuellement sous forme effervescente ou orodispersible.

D'une façon générale, la posologie sera adaptée en fonction de la voie d'administration et du patient à traiter.

L'acide lipoïque ou l'un de ses sels pharmaceutiquement acceptables peut ainsi être administré, en une, deux ou trois fois, en une quantité de 0,1 à 100 mg/kg/j, de préférence de 1 à 60 mg/kg/j et de préférence encore de 1 à 10 mg/kg/j.

L'acide hydroxycitrique, ou l'un de ses sels pharmaceutiquement acceptables, peut être administré quant à lui en une, deux ou trois fois, en une quantité de 0,1 à 100 mg/kg/j, de préférence de 1 à 50 mg/kg/j et de préférence encore de 20 à 50 mg/kg/j.

À titre d'exemple, dans l'association pharmaceutique selon l'invention, l'acide lipoïque ou l'un de ses sels pharmaceutiquement acceptables peut être présent en une quantité comprise entre 20 et 800 mg, de préférence entre 20 et 150 mg tandis que l'acide hydroxycitrique peut être présent en une quantité comprise entre 200 et 1000 mg, de préférence entre 600 et 900 mg, en vue d'une administration à raison de deux ou trois fois par jour (NB : les quantités indiquées sont calculées pour être administrées 3 fois par jour). Une association illustrative de l'invention peut ainsi comprendre 30 mg d'acide lipoïque et 800 mg d'acide hydroxycitrique dans un excipient pharmaceutiquement acceptable pour une composition à prendre trois fois par jour.

Les associations pharmaceutiques de l'invention peuvent être préparées de façon habituelle. Cette préparation comprend :
- soit la formulation des principes actifs ensemble dans un excipient pharmaceutiquement acceptable ;
- soit la formulation séparée de chacun des principes actifs dans des excipients pharmaceutiquement acceptables identiques ou non.

Les techniques de formulation susceptibles d'être utilisées à cet effet sont bien connues de l'homme du métier et comprennent essentiellement le mélange physique du (des) principe (s) actif (s) avec l' (les) excipient (s) pharmaceutiquement acceptable(s).

Compte-tenu de l'activité antitumorale élevée de l'association qui vient d'être décrite, l'invention est particulièrement utile pour la fabrication de médicaments destinés au traitement d'une maladie de la prolifération cellulaire, choisi dans le groupe comprenant les cancers du sein, des ovaires, du col, de la prostate, des testicules, de l'oesophage, de l'estomac, de la peau, des poumons, des os, du côlon, du pancréas, de la thyroïde, des voies biliaires, de la cavité buccale et du pharynx (région orale), des lèvres, de la langue, de la bouche, du pharynx, de l'intestin grêle, du côlon-rectum, du gros intestin, du rectum, du cerveau et du système nerveux central, un glioblastome, un neuroblastome, un kératoacanthome, un carcinome épidermoïde, un carcinome à grandes cellules, un adénocarcinome, un adénome, un carcinome folliculaire, un carcinome indifférencié, un carcinome papillaire, un séminome, un mélanome, un sarcome, un carcinome de la vessie, un carcinome du foie, un carcinome des reins, des troubles myéloïdes, des troubles lymphoïdes, la maladie de Hodgkin, un carcinome à tricholeucocytes et la leucémie.

Les associations pharmaceutiques de l'invention peuvent bien entendu être utilisées dans un traitement thérapeutique en complément d'autres traitements anticancéreux.

Ainsi, selon un second aspect, la présente demande vise à couvrir l'utilisation d'une association pharmaceutique telle que décrite précédemment, pour la fabrication d'un médicament à activité anti-tumorale destiné notamment au traitement des maladies précitées.

L'invention sera maintenant illustrée par les exemples suivants et par les figures annexées qui montrent respectivement :
- **Figures 1A et 1B****:** Représentation graphique des résultats des tests de viabilité cellulaire mettant en oeuvre la lignée cellulaire HT-29 traitée à l'aide de l'acide lipoïque seul (figure1A) ou de l'hydroxycitrate de calcium seul (figure 1B) ;
- **Figures 2A et 2B** **:** Représentation graphique des résultats des tests de viabilité cellulaire mettant en oeuvre la lignée cellulaire T-24 traitée à l'aide de l'acide lipoïque seul (figure2A) ou de l'hydroxycitrate de calcium seul (figure 2B) ;
- **Figures 3A à 3D** **:** Représentation graphique des résultats des tests de viabilité cellulaire mettant en oeuvre les lignées cellulaires HT-29 et T-24 traitées à l'aide d'une association d'acide lipoïque et d'hydroxycitrate de calcium selon l'invention, pour trois concentrations croissantes d'hydroxycitrate de calcium ;
- **Figure 4A** **:** Représentation graphique des résultats de traitement de souris à l'aide de l'association pharmaceutique selon l'invention.
- **Figure 4B** **:** Représentation graphique montrant la survie des souris traitées dans l'étude dont les résultats sont montrés à la figure 4A.

### EXPÉRIMENTATIONS RÉALISÉES

L'activité antitumorale remarquable de l'association thérapeutique selon l'invention a été mise en évidence par les expérimentations décrites ci-après.

### 1 - Tests sur lignées cellulaires tumorales humaines

### 1.1 Lignées cellulaires utilisées

Les lignées cellulaires tumorales humaines et les milieux de culture ont été obtenus auprès de l'ATCC (American Type Culture Collection, Manassas, Virginie, Etats-Unis).

La lignée cellulaire tumorale HT-29 a été isolée en 1964 à partir d'un adénocarcinome colique primaire sur une femme âgée de 44 ans (Fogh J. et al. 1977 J. Nat. Cancer. Inst. 59: 221-6).

La lignée cellulaire tumorale T-24 est un carcinome transitionnel de la vessie isolée par chez une femme de 81 ans (O'Toole CM. et al. 1983 Nature 301: 429-30).

### 1.2 - Conditions de culture

Les lignées cellulaires tumorales ont été cultivées en monocouche à 37°C dans une atmosphère humidifiée (5% de CO₂ 95% d'air). Le milieu de culture qui a été utilisé est du DMEM Glutamax I (Invitrogen) pour les deux lignées supplémentés avec 10% de sérum de veau foetal (Eurobio) et 1/10000 UI de pénicilline et streptomycine. Pour la réalisation des expérimentations, les lignées cellulaires tumorales humaines ont été détachées du flacon de culture par un traitement de 10 minutes avec une solution de trypsine dans du milieu de Hanks sans calcium, ni magnésium. Les cellules ont été comptées dans un hémocytomètre et leur viabilité a été déterminée par le test d'exclusion au bleu trypan.

Les lignées cellulaires tumorales ont été amplifiées puis ensemencées dans des microplaques de 96 puits (MTT) ou plaques 6 puits (comptages cellulaires) à une concentration permettant aux cellules d'être en phase de prolifération pendant les 5 jours de la culture. Elles ont été incubées pendant 48 heures avant le début des traitements dans les microplaques contenant du milieu de culture sans les substances à tester ni les substances de référence.

Au cours de la phase 1 (test avec un seul principe actif), les lignées cellulaires tumorales ont été incubées pendant 5 jours à 37°C sous 5% de CO₂ avec du milieu de culture contenant une des substances à tester. Chaque condition expérimentale a été reproduite six fois. De façon générale, les flacons de milieu de culture supplémentés avec du sérum ont été d'abord préparés avec la plus grande concentration de chaque molécule à tester. Chaque autre concentration à tester, a été obtenue par dilutions successives dans le milieu de culture supplémenté avec sérum. Cette étape a été adaptée en fonction de l'instabilité ou de la sensibilité particulière de chaque drogue.

Dans la phase II de l'étude (test avec l'association des deux principes actifs), les mêmes conditions de préparation des milieux et de culture des cellules ont été respectées et les principes actifs ont été combinés et ajoutés simultanément dans le milieu culture.

Les lignées cellulaires tumorales ont été incubées avec 100 µl (plaques 96 puits) ou 2ml (plaques 6 puits) de milieu de culture supplémenté avec sérum contenant les substances à tester ou la substance de référence. Le milieu de culture de chaque puits de culture (complété avec la ou les molécule(s) à tester) a été renouvelé tous les deux jours pendant le traitement.

### 1.3 - Test de prolifération et viabilité cellulaire

A compter du premier jour de contact et tous les deux jours après le début de mise en contact des milieux de culture contenant les substances à tester et les substances de référence aux différentes concentrations testées, la prolifération et la viabilité cellulaire des cellules a été évaluée. La viabilité cellulaire a été évaluée de deux manières différentes :
- directement, au microscope optique par un comptage statistique (nombre de cellules), et
- indirectement, par un dosage colorimétrique basé sur la dégradation de sels de tétrazolium par les déshydrogénases mitochondriales (test MTT). Les densités optiques (DO) de chaque puits ont alors été lues avec un lecteur de microplaques réglé à la longueur d'onde adaptée.

### 1.4 - Présentation des résultats

Pour chaque principe actif, chaque concentration testée et chaque combinaison, trois valeurs de viabilité cellulaire ont été mesurées et corrigées, notamment à l'aide des gammes d'étalonnage de l'appareil de lecture optique.

La moyenne des trois valeurs de mesures de viabilité cellulaire a ensuite été divisée par la moyenne des mesures de viabilité effectuée parallèlement dans une culture témoin exempt de principes actifs.

Le tableau 1 ci-après, récapitule les valeurs calculées à partir des mesures de viabilité effectuées au microscope optique ou par marquage luminescent (Nb cellules / MTT) pour la lignée cellulaire HT-29 traitée par l'acide lipoïque seul ou par l'hydroxycitrate de calcium seul.

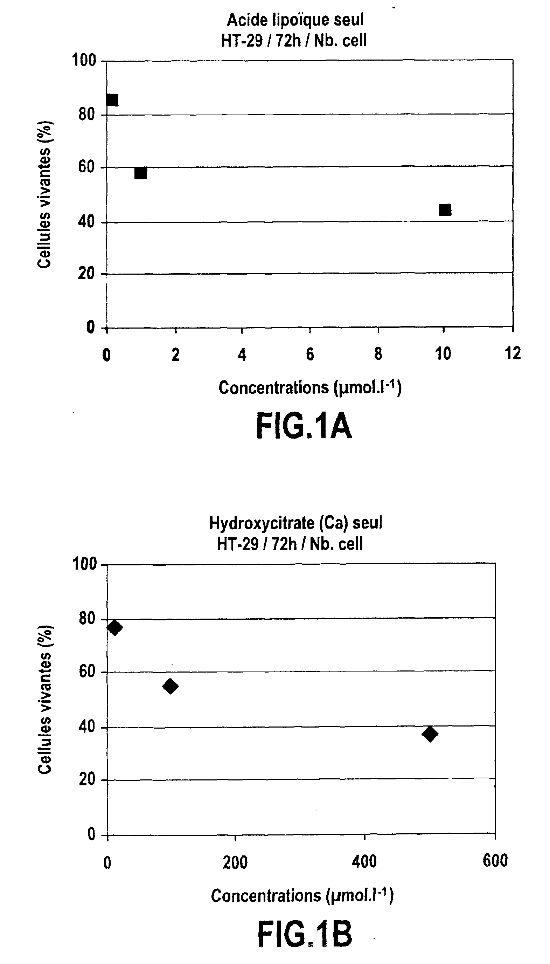

Ces valeurs ont en outre été reportées sous forme de graphiques aux figures 1A et 1B.

**Tableau 1:**

| ratio des cellules HT-29 vivantes après 72 heures | | | | |
|---|---|---|---|---|
| Molécules testées seules | | Lignée cellulaire : HT-29 Comptage réalisé après 72 heures | | |
| acide lipoïque | Concentrations (µmol.l⁻¹) | 0,1 | 1 | 10 |
| | MTT | 0,807 | 0,55 | 0,452 |
| (Fig. 1A) | Nb cellules | 0,853 | 0,583 | 0,441 |
| hydroxycitrate de calcium | Concentrations (µmol.l⁻¹) | 10 | 100 | 500 |
| | MTT | 0,743 | 0,519 | 0,376 |
| (Fig. 1B) | Nb cellules | 0,777 | 0,557 | 0,374 |

Le tableau 2 ci-après récapitule les valeurs calculées à partir des mesures de viabilité effectuées au microscope optique ou par marquage luminescent (Nb cellules / MTT) pour la lignée cellulaire T-24 traitée par l'acide lipoïque seul ou par l'hydroxycitrate de calcium seul.

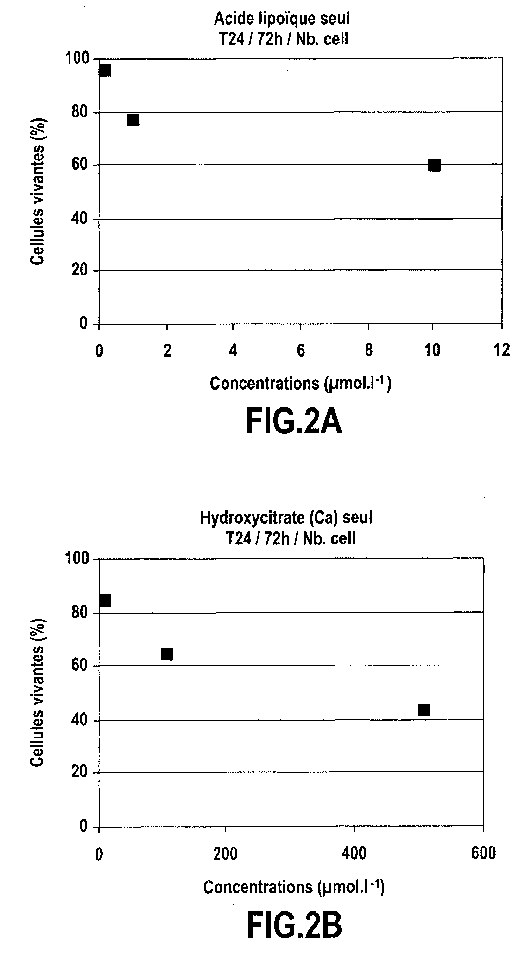

Ces valeurs ont été reportées sous forme de graphiques aux figures 2A et 2B.

Dans les graphiques des figures 1A,1B,2A,2B, l'axe des ordonnées indique le pourcentage de cellules vivantes dans chaque condition par rapport à un contrôle négatif (véhicule de dilution) comptabilisées à l'oeil nu (Nb Cell.) ou à l'aide d'un marquage des cellules vivantes (MTT) après 72 heures. L'axe des abscisses indique la concentration, exprimée en micromoles par litre, du principe actif utilisé.

**Tableau 2:**

| ratio des cellules T-24 vivantes après 72 heures | | | | |
|---|---|---|---|---|
| Molécules testées seules | | Lignée cellulaire : T-24 Comptage réalisé après 72 heures | | |
| acide lipoïque | Concentrations (µmol.l⁻¹) | 0,1 | 1 | 10 |
| | MTT | 0,898 | 0,690 | 0,534 |
| (Fig. 2A) | Nb cellules | 0,940 | 0,772 | 0,601 |
| hydroxycitrate de calcium | Concentrations (µmol.l⁻¹) | 0,2 | 3 | 45 |
| | MTT | 0,725 | 0,488 | 0,359 |
| (Fig. 2B) | Nb cellules | 0,845 | 0,643 | 0,450 |

Les tableaux 3 et 4 ci-après récapitulent les valeurs obtenues par les deux méthodes de mesure de viabilité cellulaire, en utilisant l'association des principes selon l'invention à différentes concentrations.

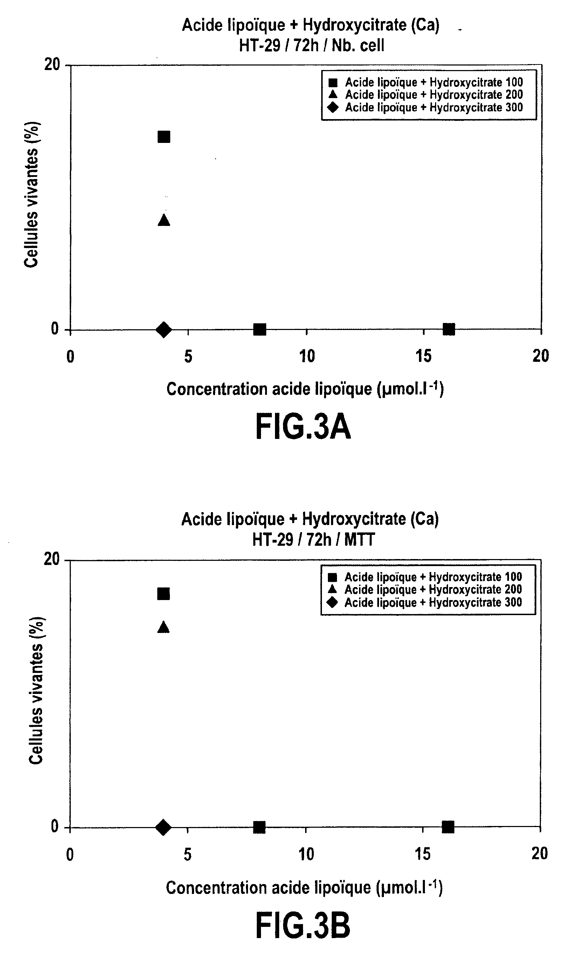

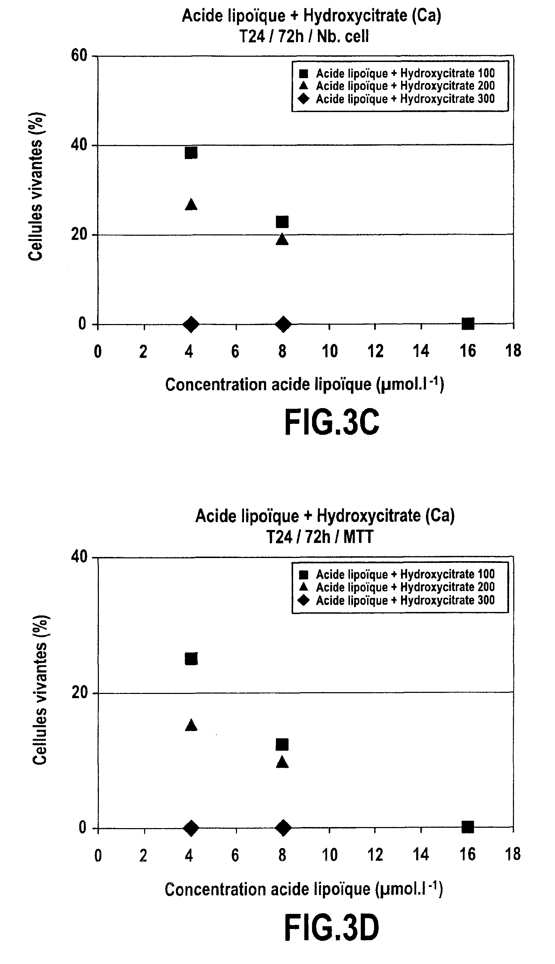

Ces valeurs ont été reportées sous forme de graphiques aux figures 3A à 3D.

Dans ces graphiques, l'axe des ordonnées indique le pourcentage de cellules vivantes dans chaque condition par rapport à un contrôle négatif (véhicule de dilution) comptabilisées à l'oeil nu (Nb Cell.) ou à l'aide d'un marquage des cellules vivantes (MTT) après 72 heures.

Les mesures de viabilité ont été effectuées à trois concentrations croissantes d' hydroxycitrate de calcium, respectivement représentées par les symboles : ■ (100 micromoles par litre), ▲ (200 micromoles par litre), et ◆. (300 micromoles par litre),

L'axe des abscisses indique la concentration, exprimée en micromoles par litre d'acide lipoïque utilisé.

**Tableau 3 :**

| résultats obtenus avec l'association acide lipoïque + hydroxycitrate (Ca) | | | | |
|---|---|---|---|---|
| Concentration hydroxycitrate (Ca) | (Fig.3B) | acide lipoïque + hydroxycitrate (Ca) | | |
| | (Fig.3A) | cellules HT-29 vivantes après 72 heures | | |
| (µmol.l⁻¹) | Concentration acide lipoïque | 4 | 8 | 16 |
| 100 | MTT | 0,177 | 0 | 0 |
| | Nb cellules | 0,147 | 0 | 0 |
| 200 | MTT | 0,154 | 0 | 0 |
| | Nb cellules | 0,085 | 0 | 0 |
| 300 | MTT | 0 | 0 | 0 |
| | Nb cellules | 0 | 0 | 0 |

**Tableau 4 :**

| résultats obtenus avec l'association acide lipoïque + hydroxycitrate (Ca) | | | | |
|---|---|---|---|---|
| Concentration hydroxycitrate (Ca) | (Fig.3D) | acide lipoïque + hydroxycitrate (Ca) | | |
| | (Fig.3C) | cellules T-24 vivantes après 72 heures | | |
| (µmol.l⁻¹) | Concentration Acide lipoïque | 4 | 8 | 16 |
| 100 | MTT | 0,229 | 0,142 | 0 |
| | Nb cellules | 0,393 | 0,220 | 0 |
| 200 | MTT | 0,163 | 0,132 | 0 |
| | Nb cellules | 0,271 | 0,195 | 0 |
| 300 | MTT | 0 | 0 | 0 |
| | Nb cellules | 0 | 0 | 0 |

### 1.5 - Commentaires des résultats :

### 1.5.1 - Principes actifs utilisés seuls

Les concentrations en principe actif qui ont été testées ont été définies en fonction des données toxicologiques disponibles pour chaque principe actif. Elles correspondent à des doses qui, dans l'hypothèse où les principes actifs seraient administrés à l'homme par voie orale, ne seraient pas toxiques.

Il est observé qu'en règle générale, ces principes actifs, ne permettent pas à eux seuls d'induire une mortalité cellulaire de 100 %, même aux concentrations testées les plus élevées.

### 1.5.2 - Principes actifs en combinaison

Il est observé que les résultats obtenus pour les deux lignées cellulaires HT-29 et T-24 sont du même ordre et que les deux méthodes de dénombrement des cellules vivantes (Nb cellules / MTT) ont des profils très proches.

L'effet de l'association d'acide lipoïque et d'hydroxycitrate sur la viabilité cellulaire des lignées HT-29 et T-24 est illustré par les graphiques des figures 3A à 3D.

Pris isolément, une concentration de 4 µmol.l⁻¹ d'acide lipoïque et de 100 µmol.l⁻¹ d'hydroxycitrate de calcium, permettent chacune, au mieux, d'induire 50% de mortalité cellulaire (figures 1A et 1B, 2A et 2B).

En revanche, lorsque l'acide lipoïque et l'hydroxycitrate sont utilisés sous forme combinée, la mortalité cellulaire dépasse 80 %. Ces résultats montrent donc un effet synergique de la combinaison des deux principes actifs.

Une mortalité cellulaire de 100% est atteinte dès que la concentration d'acide lipoïque est augmentée à 8 µmol.l⁻¹ pour une concentration d'hydroxycitrate de 200 µmol.l⁻¹.

### 2 - Tests d'activité antitumorale sur souris C3H

### 2.1 - Modèle murin

Les compositions décrites ci-après ont été testées contre les tumeurs murines de la vessie MBT-2 implantées dans des souris syngéniques C3H. Ces souris développent une tumeur de 7 à 10 mm de diamètre en environ 20 jours. Les associations pharmaceutiques selon l'invention ainsi que les compositions témoins ont été administrées par voie intrapéritonéale, durant 21 jours, à compter du 19^{eme} jour après l'inoculation des tumeurs. L'évolution du développement tumoral a été suivie par mesure de la taille des tumeurs et suivi de la survie des animaux au cours de l'expérience.

Les souris ont été randomisées en groupes de 18 individus, dont la tumeur est palpable (taille de environ 10 mm, après 19 jours environ de développement tumoral) en fonction de la taille de la tumeur et de la masse des animaux. Pour chaque animal, le diamètre maximal de chaque tumeur a été mesuré au Vernier pour déterminer le volume tumoral.

Les souris utilisées dans cette étude ont été traités conformément aux règles éthiques en vigueur.

### 2.2 - Culture et inoculation des tumeurs

La lignée cellulaire tumorale MBT-2 est un carcinome transitionnel de la vessie induit par le FANFT (*N*-[4-(5-nitro-2-furyl)-2-thiazolyl] formamide) chez une souris de lignée C3H/HeN (Soloway MS. et al 1973 Surg. Forum. 24: 542-4).

La lignée MBT-2 a été cultivée en monocouche à 37°C dans une atmosphère humidifiée (5% de CO₂ 95% d'air). Le milieu de culture qui a été utilisé est du DMEM Glutamax I (Invitrogen) supplémenté avec 10% de sérum de veau foetal (Eurobio) et 1/10000 UI de pénicilline et streptomycine.

Les cellules ont été détachées du flacon de culture par un traitement de 10 minutes avec une solution de trypsine/EDTA puis comptées.

La lignée cellulaire MBT-2 a été mise en culture à une densité de 3 x 10³ cellules/puits de 16mm d'une plaque de 24 puits, puis cultivée en monocouche à 37°C. Les cellules ont été détachées du flacon de culture par un traitement de 10 minutes avec une solution de trypsine/EDTA puis comptées.

Les cellules MBT-2 ont été dissociées dans une suspension cellulaire, et une injection a été effectuée via une aiguille de diamètre 25 gauge dans le flanc d'une souris mâle C3H de 6 semaines : 10⁶ cellules (120 µL). Le traitement a débuté dix-neuf jours après l'implantation.

### 2.3 - Combinaisons testées

L'association pharmaceutique selon l'invention a été testée in vivo:
Les produits suivants ont été utilisés pour préparer cette association :
   - Acide alpha-lipoïque (T1395 Sigma-Aldrich)
   - Hydroxycitrate de calcium (55128 Sigma-Aldrich)

Des lots de souris témoins ont été traitées à l'aide de compositions comprenant :
- un analogue de la pyridine utilisé comme médicament dans le traitement du cancer: le 5-Fluorouracile (5-FU) (Sigma F6627),
- la solution saline isotonique (9 g/L) utilisée pour l'injection intrapéritonéale sans principe actif,
- le véhicule de dissolution des principes actifs (éthanol 0,05%) (témoin neutre).

Les concentrations testées et les conditions expérimentales utilisées sont récapitulées dans les tableaux 5 et 6 suivants.

**Tableau 5 :**

| | | |
|---|---|---|
| Nombre d'injections intra-péritonéales par jour et quantités de molécules injectées en fonction des conditions. | | |

| Principes actifs | Nombre d'injection par jour | Quantité par injection |
|---|---|---|
| Acide alpha-lipdique | 2 | 10 mg/kg |
| Calcium Hydroxycitrate | 2 | 250 mg/kg |

**Tableau 6 :**

| Groupes d'animaux testées et conditions expérimentales correspondantes. | | | |
|---|---|---|---|
| Groupe | traitement | Nombre d'animaux | Durée du traitement |
| 1 | Souris naïves | 18 | - |
| 2 | Implantation milieu seul | 18 | - |
| 3 | Solution saline | 18 | 21 jours |
| 4 | Solution saline + 5-FU | 18 | 4 jours |
| 5 | Solution saline + éthanol 0,05% | 18 | 21 jours |
| 6 | Acide lipoïque et hydroxycitrate de calcium | 18 | 21 jours |

Après randomisation des animaux en différents groupes, les traitements avec les différents principes actifs ont eu lieu tous les jours par voie intrapéritonéale (IP). Les principes actifs ayant une demi-vie courte (inférieure à 12 heures), ils ont été administrés deux fois par jour (matin et soir) (cf. tableau 5).

Le traitement avec le 5-FU (contrôle positif) a été administré par voie intrapéritonéale avec une dose de 10mg/kg une fois par jour pendant 4 jours. Les souris traitées au 5-FU ont été suivies de la même façon que les autres souris.

### 2.4 - Suivi et résultats

Tous les cinq jours, l'évolution tumorale a été suivie par différentes mesures : poids des animaux, taille des tumeurs (pied à coulisse), mortalité.

Une surveillance quotidienne des animaux a été effectuée une fois par jour et a permis de déterminer avec précision le jour de la mort des animaux et de les autopsier rapidement. Cette surveillance a également permis d'isoler ou d'euthanasier les animaux faibles ou moribonds selon les recommandations de la CEE, de l'ASAB, du Canadian Council on Animal Care et l'UKCCCR.

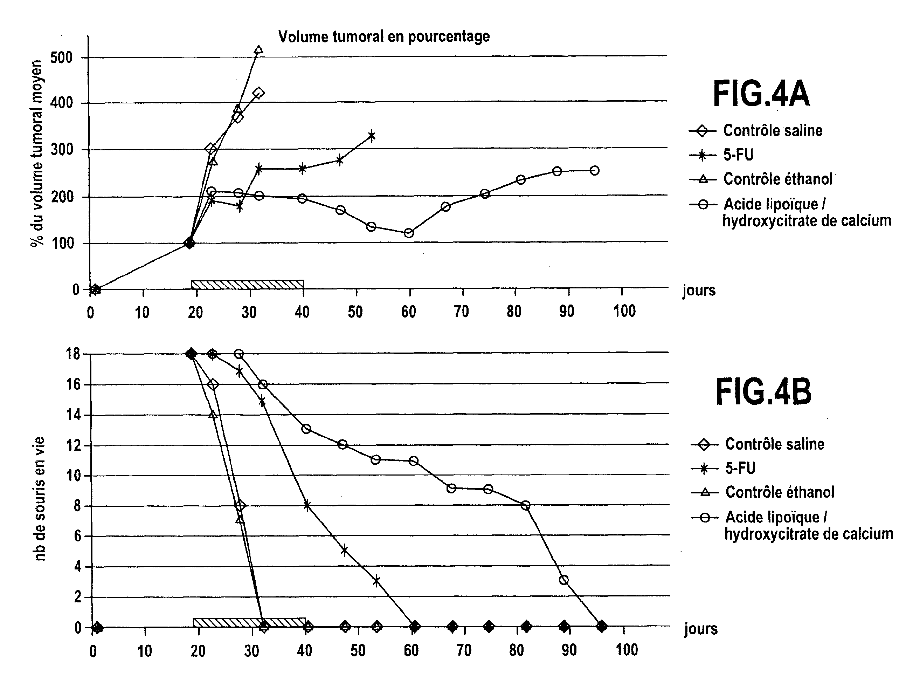

Les résultats de mesure du volume moyen des tumeurs dans chacun des groupes de souris traitées, en fonction du temps, sont reportés dans le graphique de la figure 4A.

Dans ce graphique, l'axe des ordonnées indique l'accroissement en pourcentage du volume moyen (par rapport au volume mesuré le premier jour du traitement) des tumeurs mesurées chez les souris.

L'axe des abscisses indique le nombre de jours durant lesquels les souris ont été suivies.

Les résultats montrant la survie des souris traitées dans cette étude sont reportés dans le graphique de la figure 4B.

Dans ce graphique, l'axe des ordonnées indique le nombre de souris en vie.

L'axe des abscisses indique le nombre de jours durant lesquels les souris ont été suivies.

Par ailleurs, dans les figures 4A et 4B :
- la partie grisée entre les jours 19 et 40 représente la durée pendant laquelle le traitement a été administré.
- les symboles suivants ont été utilisés:
   ○ :acide lipoïque + hydroxycitrate de calcium ;
   ◊ : contrôle solution saline ;
   * : 5-FU ;
   Δ : contrôle éthanol.

### 2.5 - Interprétation des résultats

Comme le montre le graphique de la figure 4A, l'association pharmaceutique selon l'invention permet de limiter la croissance des tumeurs, dont le volume se stabilise sur une durée d'au moins 100 jours à des valeurs de 100 % du volume atteint par les tumeurs au début du traitement.

Ce résultat est à mettre au regard du volume atteint par les tumeurs dans les souris témoins dont l'accroissement est de l'ordre de 500%.

Il est intéressant de noter que le traitement selon l'invention a permis, d'une façon tout à fait surprenante et inattendue, d'obtenir un effet de stabilisation des tumeurs significativement plus important que celui obtenu en utilisant le 5-FU, qui est un médicament anticancéreux.

Comme le montre le graphique de la figure 4B, l'association pharmaceutique selon l'invention a permis d'augmenter de façon significative le temps de survie des souris traitées.

Ainsi, dans les groupes correspondant aux contrôles solution saline et éthanol, 50% environ des souris sont encore vivantes après 27 jours d'implantation.

Dans le groupe traité à l'aide du 5-FU, 50% des souris sont encore vivantes après 39 jours d'implantation ce qui correspond à une augmentation de la survie de 12 jours.

Dans le groupe des souris traitées à l'aide de l'association pharmaceutique selon l'invention, 50% des souris sont encore vivantes après 74 jours, ce qui correspond à une augmentation de la survie de 35 jours par rapport au 5-FU (temps de survie multiplié par 1,9) et de 47 jours par rapport aux souris témoins (temps de survie multiplié par 2,7).

Ainsi, il est intéressant de noter que le traitement selon l'invention a permis, d'une façon tout à fait surprenante et inattendue, d'augmenter le taux de survie des souris de façon significativement importante par comparaison au traitement utilisant le 5-FU.

### 3 - Résultats obtenus avec d'autres principes actifs additionnels

Des essais complémentaires ont été réalisés afin d'évaluer l'efficacité d'une association pharmaceutique incorporant l'acide lipoïque ou l'un de ses sels pharmaceutiquement acceptables ; l'acide hydroxycitrique ou l'un de ses sels pharmaceutiquement acceptables et au moins un principe actif additionnel, notamment un principe actif à activité anti-tumorale.

Ces expériences complémentaires ont mis en évidence le fait que l'efficacité d'une association d'acide lipoïque (ou d'un de ses sels pharmaceutiquement acceptables) et d'acide hydroxycitrique (ou d'un de ses sels pharmaceutiquement acceptables) peut être encore améliorée lorsque ces deux principes actifs sont associés à un principe actif additionnel notamment choisi dans le groupe constitué du cisplatine, de la capsaïcine, de la choline, de la miltéfosine et de la vitamine B12.

Ces essais ont été réalisés sur les modèles MBT-2 (carcinome de la vessie) ou LL/2 (carcinome pulmonaire) implantés dans le dos de souris syngéniques (9 souris/groupe).

Lorsque le volume tumoral a atteint environ 100 mm³, les souris ont été traitées par voie intrapéritonéale pendant 3 semaines selon les doses suivantes :
- acide hydroxycitrique (ci-après HCA) 250 mg/kg deux fois par jour,
- acide lipoïque (ci-après ALA) 10 mg/kg deux fois par jour,
- cisplatine 1 mg/kg un jour sur deux,
- capsaïcine 5 mg/kg ou 750 µg/kg une fois par jour,
- miltéfosine 20 mg/kg/jour,
- vitamine B12 5 µg/kg/jour.

Le développement tumoral a été suivi par une mesure régulière des dimensions de la tumeur et l'inhibition de la croissance tumorale a été calculé par le rapport T/C% (ratio entre le volume tumoral moyen du groupe traité par rapport au groupe contrôle à un temps donné).
À l'issue de ces tests il a été observé que :
- L'utilisation d'une association de ALA, HCA et cisplatine permet de réduire de 40% le développement tumoral par rapport à l'association ALA/HCA seule (modèle MBT-2).
- L'utilisation d'une association de ALA, HCA et capsaïcine (5mg/kg/jour) permet de réduire de 66% le développement tumoral par rapport à l'association ALA/HCA seule (modèle LL/2).
- L'utilisation d'une association de ALA, HCA, capsaïcine (750pg/kg/jour) et cisplatine permet de réduire de 32% le développement tumoral par rapport à l'association ALA/HCA seule (modèle LL/2).
- L'utilisation d'une association de ALA, HCA, capsaïcine, miltéfosine, choline et vitamine B12 permet de réduire de 21% le développement tumoral par rapport à l'association ALA/HCA seule (modèle LL/2).

### Exemple de composition pharmaceutique selon l'invention

Une composition pharmaceutique selon l'invention peut être, par exemple, formulée sous forme de gélule contenant les ingrédients suivants :
50 mg d'acide alpha lipoïque
400 mg d'acide hydroxycitrique
109 mg d'une enveloppe constituée d'hydroxypropylméthyl-cellulose
20 mg d'anti-agglomérants, association de stéarate de magnésium végétal et de dioxyde de silicium
15 mg d'agent liant (hydroxypropyl cellulose).

Une telle composition peut être administrée selon cette posologie à raison de 2 gélules, 3 fois par jour, au minimum une heure avant les repas.

## Revendications

1. Association pharmaceutique, **caractérisée en ce qu'**elle comprend, à titre de principes actifs :
- l'acide lipoïque ou l'un de ses sels pharmaceutiquement acceptables ; et
- l'acide hydroxycitrique ou l'un de ses sels pharmaceutiquement acceptables ;
lesdits ingrédients actifs étant formulés ensemble ou séparément, pour une utilisation conjuguée, simultanée ou séparée, dans le traitement des tumeurs.

2. Association pharmaceutique pour utilisation selon la revendication 1, **caractérisée en ce que** le sel de l'acide hydroxycitrique précité est un sel de métal alcalin ou alcalino-terreux.

3. Association pharmaceutique pour utilisation selon la revendication 1 ou 2, **caractérisée en ce que** le sel de l'acide hydroxycitrique précité est le sel de calcium.

4. Association pharmaceutique pour utilisation selon l'une quelconque des revendications 1 à 3, **caractérisée en ce que** les principes actifs précités se présentent :
- ensemble, sous une forme galénique adaptée à une administration orale ; ou
- séparément, indépendamment l'un de l'autre, chacun sous une forme galénique adaptée à l'administration par voie orale.

5. Association pharmaceutique pour utilisation selon la revendication 4, **caractérisée en ce que** la forme galénique adaptée à la voie orale précitée est choisie parmi les comprimés, les gélules, les poudres, les granulés, les liophilisats, les solutés buvables et les sirops.

6. Association pharmaceutique pour utilisation selon l'une quelconque des revendications 1 à 5, **caractérisée en ce qu'**elle se présente sous une forme unitaire dans laquelle les principes actifs précités, ensemble, se présentent sous forme de comprimés.

7. Association pharmaceutique pour utilisation selon l'une quelconque des revendications 1 à 6, **caractérisée en ce qu'**elle comprend :
- l'acide lipoïque ou l'un de ses sels pharmaceutiquement acceptables en une quantité comprise entre 20 et 800 mg, de préférence entre 20 et 150 mg ;
- l'acide hydroxycitrique ou l'un de ses sels pharmaceutiquement acceptables en une quantité comprise entre 200 et 1000 mg, de préférence entre 600 et 900 mg.

8. Association pharmaceutique pour utilisation selon l'une quelconque des revendications 1 à 7, **caractérisée en ce qu'**elle comprend au moins un autre principe actif.

9. Association pharmaceutique pour utilisation selon l'une quelconque des revendications 1 à 8, **caractérisée en ce qu'**elle comprend au moins un principe actif additionnel choisi dans le groupe constitué du cisplatine, de la capsaïcine, de la choline, de la miltéfosine et de la vitamine B12.

10. Association pharmaceutique pour utilisation selon la revendication 9, **caractérisée en ce qu'**elle comprend un principe actif additionnel choisi parmi le cisplatine et la capsaïcine.

11. Association pharmaceutique pour utilisation selon la revendication 9, **caractérisée en ce qu'**elle comprend les deux principes actifs additionnels suivants : cisplatine et capsaïcine.

12. Association pharmaceutique pour utilisation selon la revendication 9, **caractérisée en ce qu'**elle comprend les quatre principes actifs additionnels suivants : capsaïcine, vitamine B12, choline et miltéfosine.

13. Utilisation d'une association pharmaceutique selon l'une quelconque des revendications 1 à 12, pour la fabrication d'un médicament à activité anti-tumorale.

## Claims

1. A pharmaceutical combination, **characterized in that** it comprises, as active ingredients:
- lipoic acid or one of the pharmaceutically acceptable salts thereof; and
- hydroxycitric acid or one of the pharmaceutically acceptable salts thereof;
said active ingredients being formulated together or separately, for a conjugated, simultaneous or separate use, as a composition having an antitumor activity.

2. The pharmaceutical combination for use according to claim 1, **characterized in that** the above-mentioned hydroxycitric acid salt is an alkali metal or alkaline-earth metal salt.

3. The pharmaceutical combination for use according to claim 1 or 2, **characterized in that** the above-mentioned hydroxycitric acid salt is the calcium salt.

4. The pharmaceutical combination for use according to any one of claims 1 to 3, **characterized in that** the above-mentioned active ingredients are provided:
- together, in a dosage form suitable for oral administration; or
- separately, independently of one another, each in a dosage form suitable for oral administration.

5. The pharmaceutical combination for use according to claim 4, **characterized in that** the above-mentioned dosage form suitable for oral administration is chosen from tablets, gelatin capsules, powders, granules, lyophilisates, oral solutes and syrups.

6. The pharmaceutical combination for use according to any one of claims 1 to 5, **characterized in that** it is in the form of single dosage units in which the above-mentioned active ingredients, together, are in the form of tablets.

7. The pharmaceutical combination for use according to any one of claims 1 to 6, **characterized in that** it comprises:
- lipoic acid or one of the pharmaceutically acceptable salts thereof in an amount of between 20 and 800 mg, preferably between 20 and 150 mg;
- hydroxycitric acid or one of the pharmaceutically acceptable salts thereof in an amount of between 200 and 1000 mg, preferably between 600 and 900 mg.

8. The pharmaceutical combination for use according to any one of claims 1 to 7, **characterized in that** it comprises at least on additional active ingredient.

9. The pharmaceutical combination for use according to any one of claims 1 to 8, **characterized in that** it comprises at least one additional active ingredient chosen from the group consisting of cisplatin, capsaicin, choline, miltefosine and vitamin B12.

10. The pharmaceutical combination for use according to claim 9, **characterized in that** it comprises an additional active ingredient chosen from cisplatin and capsaicin.

11. The pharmaceutical combination for use according to claim 9, **characterized in that** it comprises the following two additional active ingredients: cisplatin and capsaicin.

12. The pharmaceutical combination for use according to claim 9, **characterized in that** it comprises the following four additional active ingredients: capsaicin, vitamin B12, choline and miltefosine.

13. The use of a pharmaceutical combination as claimed in any one of claims 1 to 12, for producing a medicament having an antitumor activity.

## Patentansprüche

1. Pharmazeutische Verbindung, **dadurch gekennzeichnet, daß** sie als Wirkstoffe umfaßt:
- Liponsäure oder eines ihrer pharmazeutisch akzeptablen Salze und
- Hydroxycitronensäure oder eines ihrer pharmazeutisch akzeptablen Salze, wobei die Wirkbestandteile gemeinsam oder getrennt formuliert werden, für eine gekoppelte, gleichzeitige oder getrennte Verwendung bei der Behandlung von Tumoren.

2. Pharmazeutische Verbindung zur Verwendung, nach Anspruch 1, **dadurch gekennzeichnet, daß** das vorgenannte Salz der Hydroxycitronensäure ein Alkalimetall- oder Erdalkalimetallsalz ist.

3. Pharmazeutische Verbindung zur Verwendung, nach Anspruch 1 oder 2, **dadurch gekennzeichnet, daß** das vorgenannte Salz der Hydroxycitronensäure Calciumsalz ist.

4. Pharmazeutische Verbindung zur Verwendung, nach einem der Ansprüche 1 bis 3, **dadurch gekennzeichnet, daß** die vorgenannten Wirkstoffe:
- gemeinsam vorliegen, in einer für eine orale Verabreichung geeigneten galenischen Form oder
- unabhängig voneinander getrennt vorliegen, jeweils in einer für die Verabreichung auf oralem Weg geeigneten galenischen Form.

5. Pharmazeutische Verbindung zur Verwendung, nach Anspruch 4, **dadurch gekennzeichnet, daß** die vorgenannte für den oralen Weg geeignete galenische Form aus Tabletten, Kapseln, Pulvern, Granulat, Lyophilisaten, gelösten Substanzen zum Einnehmen und Sirups ausgewählt ist.

6. Pharmazeutische Verbindung zur Verwendung, nach einem der Ansprüche 1 bis 5, **dadurch gekennzeichnet, daß** sie in einer einheitlichen Form vorliegt, bei der die vorgenannten Wirkstoffe gemeinsam in Form von Tabletten vorliegen.

7. Pharmazeutische Verbindung zur Verwendung, nach einem der Ansprüche 1 bis 6, **dadurch gekennzeichnet, daß** sie umfaßt:
- Liponsäure oder eines ihrer pharmazeutisch akzeptablen Salze in einer Menge zwischen 20 und 800 mg, vorzugsweise zwischen 20 und 150 mg,
- Hydroxycitronensäure oder eines ihrer pharmazeutisch akzeptablen Salze in einer Menge zwischen 200 und 1000 mg, vorzugsweise zwischen 600 und 900 mg.

8. Pharmazeutische Verbindung zur Verwendung, nach einem der Ansprüche 1 bis 7, **dadurch gekennzeichnet, daß** sie wenigstens einen weiteren Wirkstoff umfaßt.

9. Pharmazeutische Verbindung zur Verwendung, nach einem der Ansprüche 1 bis 8, **dadurch gekennzeichnet, daß** sie wenigstens einen zusätzlichen Wirkstoff umfaßt, der aus der Gruppe bestehend aus Cisplatin, Capsaicin, Cholin, Miltefosin und Vitamin B12 ausgewählt ist.

10. Pharmazeutische Verbindung zur Verwendung, nach Anspruch 9, **dadurch gekennzeichnet, daß** sie einen zusätzlichen Wirkstoff umfaßt, der aus Cisplatin und Capsaicin ausgewählt ist.

11. Pharmazeutische Verbindung zur Verwendung, nach Anspruch 9, **dadurch gekennzeichnet, daß** sie die beiden folgenden zusätzlichen Wirkstoffe umfaßt: Cisplatin und Capsaicin.

12. Pharmazeutische Verbindung zur Verwendung, nach Anspruch 9, **dadurch gekennzeichnet, daß** sie die vier folgenden zusätzlichen Wirkstoffe umfaßt: Capsaicin, Vitamin B12, Cholin und Miltefosin.

13. Verwendung einer pharmazeutischen Verbindung nach einem der Ansprüche 1 bis 12, für die Herstellung eines Medikaments mit antitumoraler Aktivität.
